(19) Europäisches Patentamt
European Patent Office
Office européen des brevets

(11) Publication number: **0 185 429**
**A1**

(12) # EUROPEAN PATENT APPLICATION

(21) Application number: 85202081.7

(22) Date of filing: 16.12.85

(51) Int. Cl.⁴: C 07 D 319/20
C 07 D 263/58, C 07 D 249/18
A 61 K 31/495

(30) Priority: 21.12.84 NL 8403918

(43) Date of publication of application:
25.06.86 Bulletin 86/26

(84) Designated Contracting States:
AT BE CH DE FR GB IT LI LU NL SE

(71) Applicant: DUPHAR INTERNATIONAL RESEARCH B.V
C.J. van Houtenlaan 36
NL-1381 CP Weesp(NL)

(72) Inventor: Hartog, Jan c/o OCTROOIBUREAU ZOAN B.V.
Apollolaan 151
NL-1077 AR Amsterdam(NL)

(72) Inventor: Wouters, Wouter c/o OCTROOIBUREAU ZOAN B.V.
Apollolaan 151
NL-1077 AR Amsterdam(NL)

(72) Inventor: van Wijngaarden, Ineke c/o OCTROOIBUREAU ZOAN B.V.
Apollolaan 151
NL-1077 AR Amsterdam(NL)

(74) Representative: Muis, Maarten et al,
OCTROOIBUREAU ZOAN B.V. P.O. Box 140
NL-1380 AC Weesp(NL)

(54) **New bicyclic heteroaryl piperazines.**

(57) The invention relates to a group of new bicyclic heteroarylpiperazine derivatives which can be used as starting materials for the preparation of other compounds. Moreover, some of the compounds have interesting blood-pressure lowering activity.

The compounds can be prepared according to methods known for the preparation of analogous compounds.

EP 0 185 429 A1

Croydon Printing Company Ltd

New bicyclic heteroarylpiperazines.

The invention relates to new bicyclic heteroarylpiperazine derivatives which can be used as starting materials for the production of among others new and known piperazine derivatives having antipsychotic properties as described in a copending patent application DIR 0360. Moreover, it has been found that some of the bicyclic heteroarylpiperazine derivatives have a very interesting blood-pressure lowering activity.

It has been found that compounds of the formula 1

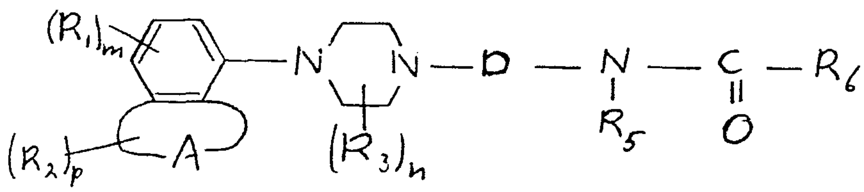

(1)

wherein

-A together with the two carbon atoms of the phenyl group forms an entirely or partly unsaturated cyclic group having 5-7 ring atoms with in the ring 1-3 hetero atoms from the group O, S and N, with the proviso that the sum of the number of oxygen atoms and sulphur atoms is at most 2, and that the nitrogen atoms in the ring may be substituted with a group $R_4$ which may be hydrogen, alkyl, hydroxyalkyl or acyl;

-$R_1$ and $R_2$ may be alkyl, cycloalkyl, optionally substituted phenyl or heteroaryl, hydroxyalkyl, alkoxyalkyl, alkoxy, aryloxy, alkylthio, arylthio, mono- or dialkylamino, mono- or diarylamino, hydroxyl, amino, alkyl-, alkoxy- or amino, or mono- or dialkylaminocarbonyl, nitro, cyano, halogen, trifluoromethyl, trifluoromethoxy, alkyl- or amino- or mono- or dialkylaminosulphonyl; $R_2$ may moreover be an oxo group

or thioxo group; m has the value 0-3 and p has the value 0-2;

$-R_3$ is an alkyl group and n has the value 0-2;

-D is an optionally branched alkylene chain,

$-R_5$ is hydrogen, and

$-R_6$ is alkyl, cycloalkyl, a phenyl group optionally substituted with a group $R_1$, in which $R_1$ has the above-mentioned meaning, or $R_6$ is a saturated or unsaturated heterocyclic group,

are very useful as starting materials for the preparation of phenylpiperazine derivatives of formula (1) in which $R_5$ is a substituent for example alkyl.

Further it has been found that the compounds of formula (1), wherein A, $R_2$, $R_3$, $R_5$, $R_6$, n and p have the above mentioned meanings,

$-R_1$ is fluoro and m is 0 or 1, and

-D is a straight or branched alkylene chain of 2-10 carbon atoms have very good blood-pressure lowering properties.

On the basis of their properties the following compounds are to be preferred in particular:

1) (+)-4-fluoro-N-[3-[4-[5-(2-hydroxymethyl-1,4-benzodioxanyl)]-1-piperazinyl]propyl]benzamide,
2) (+)-4-fluoro-N-[4-[4-[5-(2-hydroxymethyl-1,4-benzodioxanyl)]-1-piperazinyl]butyl]benzamide,
3) 4-fluoro-N-[3-[4-[7-(2-hydroxybenzoxazolyl)]-1-piperazinyl]propyl]benzamide,
4) 4-fluoro-N-[3-[4-(4-benzotriazolyl)-1-piperazinyl]propyl]benzamide.

The invention also includes the so-called prodrugs and acid addition salts of the blood-pressure lowering active compounds of formula 1. Prodrugs are to be understood to mean derivatives which are inactive as such, and which, after administration are converted in the body into an active compound of formula 1.

Suitable acids with which the compounds according to the invention can form pharmaceutically acceptable salts

are, for example, hydrochloric acid, sulfuric acid, phosphoric acid, nitric acid, organic acids such as citric acid, fumaric acid, tartaric acid, acetic acid, maleic acid, benzoic acid, p-toluenesulfonic acid, methanesulphonic acid, and the like.

When the compounds according to the invention comprise one or more optically active carbon atoms, both the racemates and the individual enantiomers belong to the invention.

The compounds according to the invention are useful as agents for circulatory diseases. They can be used as blood-pressure lowering agents. In general the compounds have a long duration of action. The compounds have no or substantially no sedating properties and substantially no influence on the heart frequency.

The blood-pressure lowering activity of the compounds according to the invention could be measured in at least one of the two following test models.

A. Blood-pressure measurement in the normotensive anaesthetized cat.

In this model the hypotension is measured in normotensive cats which are anaesthetized with 35 mg/kg (i.p.) of pentobarbital. The animals are provided with an arterial canule in order to be able to record the blood pressure directly and continuously. The compound to be tested, dissolved in a physiological saline solution or in another suitable carrier, is administered intravenously in increasing doses. The maximum effect on the mean blood pressure, the heart frequency which is derived from the blood pressure pulse, and the duration of these effects are measured.

The effectiveness of the tested compound is represented as $ED_{80}$, i.e. the dosis determined by extrapolation at which 20% decrease of the mean blood pressure occurs.

B. Blood pressure measurement in the spontaneoulsy hypertensive rat (SHR).

Spontaneously hypertensive rats are used in this model. The animals are provided with an arterial canule while under a light ether anaesthesia. At least two days later the animals are used for the blood pressure test. The mean arterial blood pressure is measured continuously. The compound to be tested is suspended in tragacanth and administered through a stomach tube. The maximum decrease of the blood pressure and the duration of the effect are measured. The $ED_{80}$ is calculated from the found values for the various dosages.

In model A the compounds according to the invention have an $ED_{80}$ which is between $10^{-4}$ and 1 mg/kg and/or in model B they have an $ED_{80}$ between about 1 and 25 mg/kg.

The compounds can be brought in a form of administration which is suitable for human application as an blood--pressure lowering agent, i.e. they may be formulated to compositions which are suitable for this purpose and can preferably be administered orally.

The new compounds according to the invention can be obtained in a manner known for the synthesis of analogous compounds.

The compounds of formula (1) can be obtained, for example, by reaction of a compound of formula 2

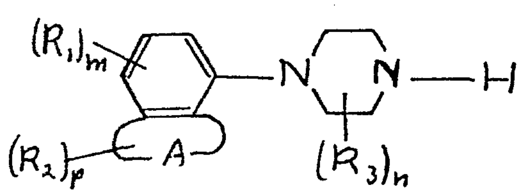

(2)

with a compound of the formula

$$L - D - NR_5 - CO - R_6,$$

in which formulae $R_1$-$R_3$, $R_5$, m, n, p, A, D, and $R_6$ have the above-mentioned meanings, and L is a so-called "leaving" group, preferably chlorine, bromine or tosylate.

This reaction may be carried out both with and without an inert organic solvent. Suitable organic solvents are, for example, methyl ethyl ketone, dimethylformamide, tetrahydrofuran, petroleum ether, alcohol and acetonitrile. In order to bind the releasing acid, an acid binder, for example $NaHCO_3$ or $K_2CO_3$ may be used. The reaction temperature is usually between room temperature and the boiling-point of the solvent used.

The starting substances of formula 2 in which the symbols have the meanings mentioned above, can be prepared according to methods described, for example, in Netherlands Patent Application 80.05133 and 82.01708.

Further compounds of formula (1) can be obtained:

a) by reacting a compound of formula (3)

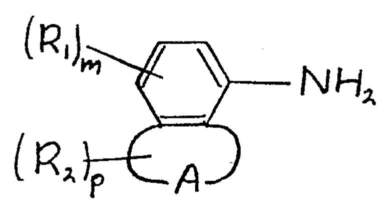

(3)

with a compound of formula 4

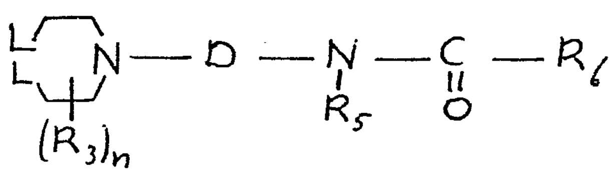

(4)

The symbols in these formulae have the above-mentioned meanings, and L is a "leaving" group, for example halogen, preferably chlorine.

b) by reacting a compound $L-CH_2-CH_2-L$ with a compound of formula 5

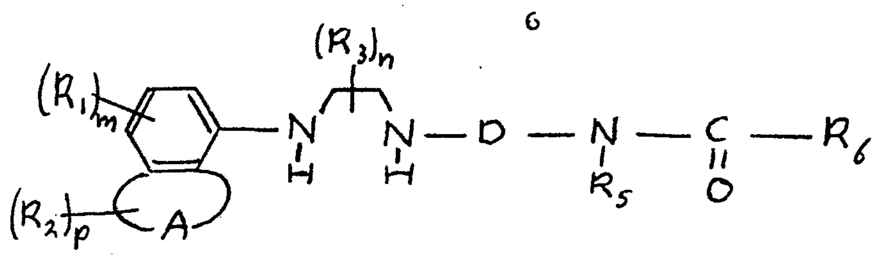

(5)

in which the symbols have the above-mentioned meanings.

Other methods, known for analogous compounds <u>inter alia</u> from Netherlands Patent Application 80.05133, which may be used for the preparation of the compounds according to the invention are:

a) Reaction of a compound of formula 6

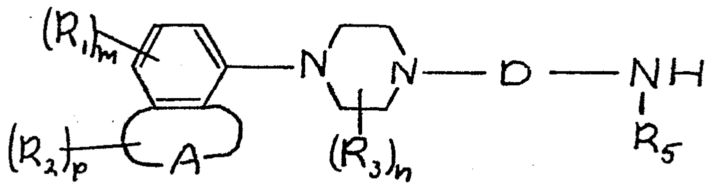

(6)

with

1) an ester of an acid of the formula $HOOC-R_6$, or

2) an acid chloride of the formula $Cl-CO-R_6$, or

3) a mixed anhydride of the formula $R_4-O-CO-O-CO-R_6$, or

4) a so-called Mukayama ester of formula 7

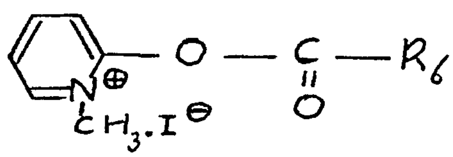

(7)

The symbols in these formulae have the above-mentioned meanings, and $R_4$ is preferably an alkyl group having 1-3 carbon atoms.

b) Reaction of a compound of formula 8

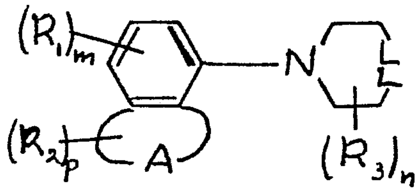

(8)

with a compound of the formula $NH_2$-D-$NR_5$-CO-$R_6$, wherein the symbols have the meanings given hereinbefore.

Furthermore, the compounds of the formula (1), wherein A is a group of the formula -N-$CR_2$=N- or -NH-N=N- and the remaining symbols have the above mentioned meanings can be obtained by reacting a compound of the formula (9)

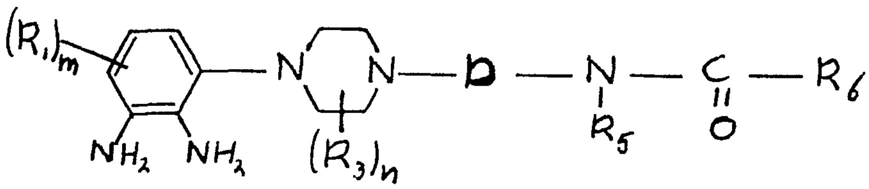

(9)

a) with an ester of an imino acid of the formula (10)

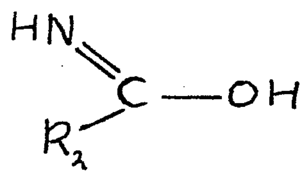

(10)

or

b) with a carboxylic acid of the formula $R_2$-COOH, or

c) with a reactive carbonyl compound, preferably, N,N'--carbonyldiimidazole, or

d) with an alkali metal nitrite in an aqueous medium, preferably sodium nitrite, in the presence of hydrochloric acid.

Further, the compounds in which $R_2$ is a hydroxyalkyl group can be obtained by hydrolysis of the corresponding compound in which $R_2$ is an esterified hydroxyalkyl group. Also, conversely, compounds in which $R_2$ is a hydroxyalkyl group can be converted into final products in which $R_2$ is an esterified hydroxyalkyl group, by esterification in a way known per se.

Finally, the desired final products of formula 1 in which the symbols have the meanings given hereinbefore, can be obtained in that, as the last reaction step, one or

more protective groups used for the protection of functional groups are removed or converted by means of methods usual for this purpose.

The individual enantiomers of compounds of formula 1 can be obtained according to methods known per se, for example, by starting from optically active intermediate products, or by resolving the racemic end products into the enantiomers by means of the methods usual for this purpose.

The invention will now be described in greater detail with reference to the following specific examples.

EXAMPLE I

(+)-4-Fluoro-N-[3-[4-[5-(2-hydroxymethyl-1,4-benzodioxanyl)]-1-piperazinyl]propyl]benzamide, dihydrochloride.

4.63 Mmol (1.42 g) of 3-[4-[5-(2-hydroxymethyl-1-1,4--benzodioxanyl)]-1-piperazinyl]propylamine and 9.26 mmol (0.94 g) of triethylamine were dissolved in 20 ml of methylene chloride. While cooling with ice, a solution of 9.26 mmol (1.47 g) of 4-fluorobenzoylchloride in 10 ml methylene chloride was added in 20 minutes while stirring was continued for one hour at 5°C and 16 hours at room temperature. The mixture was than extracted twice with 20 ml of water, and twice with 15 ml of 2N sodium hydroxide and twice with 20 ml of brine solution. After drying on magnesium sulphate, the organic layer was evaporated to dryness in vacuo.

The crude 4-fluoro-N-[3-[4-[5-[2-(4-fluorobenzoyloxymethyl)-1,4-benzodioxanyl]]-1-piperazinyl]propyl]benzamide was dissolved in 60 ml of ethanol.

4.64 Mmol (0.26 g) of potassiumhydroxide in 10 ml of water were added at once. After stirring at room temperature for 2 hours the reaction mixture was evaporated to dryness in vacuo. The resulting residue was dissolved in

50 ml of methylene chloride and washed successively with 50 ml of water and with 50 ml of a 5% sodium bicarbonate solution.

The solution was dried on magnesium sulphate and then evaporated to dryness in vacuo.

The residue was chromatographed over silicagel with methylene chloride-methanol (95-5) as an eluent.

The resulting base was dissolved in ethanol and after addition of 2 equivalents of hydrochloric acid in ethanol, the solution was diluted with ether, after which the title compound was isolated having a melting-point of 183-185°C.

The following compounds were prepared from the indicated starting materials in an analogous manner:

1) (+)-4-fluoro-N-[4-[4-[5-(2-hydroxymethyl-1,4-benzodioxanyl)]-1-piperazinyl]butyl]benzamide, dihydrochloride; melting point 206-209°C (ethanol-ether 1:1) from (+)-4-[4-[5-(2-hydroxymethyl-1,4-benzodioxanyl)]-1-piperazinyl]butylamine and 4-fluorobenzoylchloride.

2) (+)-4-fluoro-N-[5-[4-[5-(2-hydroxymethyl-1,4-benzodioxanyl)]-1-piperazinyl]pentyl]benzamide, dihydrochloride; melting point 183-187°C (ethanol/ether 3:1) from (+)-5-[4-[5-(2-hydroxymethyl-1,4-benzodioxanyl)]-1-piperazinyl]pentylamine and 4-fluorobenzoylchloride.

3) (+)-4-fluoro-N-[7-[4-[5-(2-hydroxymethyl-1,4-benzodioxanyl)]-1-piperazinyl]heptyl]benzamide, dihydrochloride; melting point 174-182°C decomposition (ethanol/ethylacetate 1:1) from (+)-7-[4-[5-(2-hydroxymethyl-1,4-benzodioxanyl)]-1-piperazinyl]heptylamine and 4-fluorobenzoylchloride.

## EXAMPLE II

### 4-Fluoro-N-[3-[4-[7-(2-hydroxybenzoxazolyl)]-1-piperazinyl]propyl]benzamide.

6.67 Mmol (1,84 g) of 3-[4-[7-(2-hydroxybenzoxazolyl)]-1-piperazinyl]propylamine were suspended in 100 ml of methylene chloride. A solution of 6,67 mmol (1,06 g) of 4-fluorobenzoylchloride in 10 ml of methylene chloride was added dropwise, while stirring.

After stirring at room temperature for 16 hours the reaction mixture was evaporated to dryness under reduced pressure. The residue was dissolved in 40 ml of methanol and the resulting solution was refluxed for 1 hour. After evaporation to dryness under reduced pressure, the product was isolated by chromatography over silicagel with a mixture of methylene chloride, methanol and ammonia as an eluent. After crystallization from 35 ml of ethanol the title compound was obtained having a melting point of 215-218°C.

EXAMPLE III

4-Fluoro-N-[3-[4-(4-benzotriazolyl)-1-piperazinyl]propyl]-benzamide.

4.65 Mmol (1.72 g) of 4-fluoro-N-[3-[4-(2,3-diaminophenyl)-1-piperazinyl]propyl]benzamide were dissolved in 10 ml of acetic acid and 10 ml of water. 5.1 Mmol (0.55 g) of sodium nitrite dissolved in 10 ml of water were added at 0-5°C to the solution over a period of 90 minutes. Then the mixture was stirred for 2 hours at room temperature, and the solution was evaporated to dryness. 15 Ml of 4N ammonia were added to the residue followed by extraction with methylene chloride. The organic layer was evaporated to dryness under reduced pressure, and the residue was chromatographed over silicagel with a mixture of methylene chloride, methanol and 25% of ammonia (92:7.5:2.5) as the eluent. The so obtained free base was crystallized from a mixture of ethanol and ether (1:6), giving the title compound with a melting-point of 204-205°C.

CLAIMS

1. Compounds of formula (1)

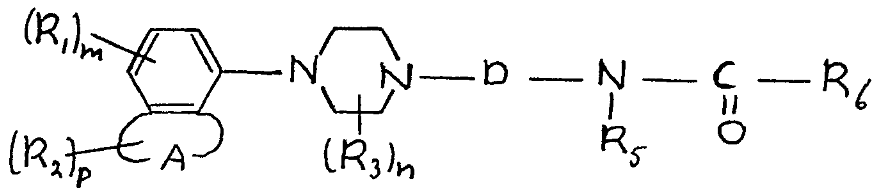

(1)

wherein

-A together with the two carbon atoms of the phenyl group forms an entirely or partly unsaturated cyclic group having 5-7 ring atoms with in the ring 1-3 hetero atoms from the group O, S and N, with the proviso that the sum of the number of oxygen atoms and sulphur atoms is at most 2, and that the nitrogen atoms in the ring may be substituted with a group $R_4$ which may be hydrogen, alkyl, hydroxyalkyl or acyl;

-$R_1$ and $R_2$ may be alkyl, cycloalkyl, optionally substituted phenyl or heteroaryl, hydroxyalkyl, alkoxyalkyl, alkoxy, aryloxy, alkylthio, arylthio, mono- or dialkylamino, mono- or diarylamino, hydroxyl amino, alkyl-, alkoxy- or amino, or mono- or dialkylaminocarbonyl, nitro, cyano, halogen, trifluoromethyl, trifluoromethoxy, alkyl- or amino- or mono- or dialkylaminosulphonyl; $R_2$ may moreover be an oxo group or thioxo group; m has the value 0-3 and p has the value 0-2; and

-$R_3$ is an alkyl group and n has the value 0-2,

-D is an optionally branched alkyl chain,

-$R_5$ is hydrogen, and

-$R_6$ is alkyl, cycloalkyl, a phenyl group substituted with a group $R_1$, in which $R_1$ has the above-mentioned meaning, or $R_6$ is a saturated or unsaturated heterocyclic group,

and acid addition salts and prodrugs thereof.

2. Blood-pressure lowering compounds of the formula 1 as claimed in Claim 1, characterized in that A, $R_2$, $R_3$, $R_5$, $R_6$, n and p have the meaning given in Claim 1, $R_1$ is fluoro, m is 0 or 1, and D is a straight or branched alkylene chain of 2-10 carbon atoms, and acid addition salts and prodrugs thereof.

3. Compounds:

a) (+)-4-fluoro-N-[3-[4-[5-(2-hydroxymethyl-1,4-benzodioxanyl)]-1-piperazinyl]propyl]benzamide,

b) (+)-4-fluoro-N-[4-[4-[5-(2-hydroxymethyl-1,4-benzodioxanyl)]-1-piperazinyl]butyl]benzamide,

c) 4-fluoro-N-[3-[4-[7-(2-hydroxybenzoxazolyl)]-1-piperazinyl]propyl]benzamide,

d) 4-fluoro-N-[3-[4-(4-benzotriazolyl)-1-piperazinyl]propyl]benzamide.

4. Pharmaceutical composition having blood-pressure lowering activity, characterized in that it contains at least one compound as claimed in Claim 2 therein as active component.

5. A method of preparing blood-pressure lowering compositions as claimed in Claim 4, characterized in that a compound as claimed in Claim 2 is brought into a form suitable for administration.

6. A method of preparing compounds as claimed in Claim 1, characterized in that a compound of the formula (2)

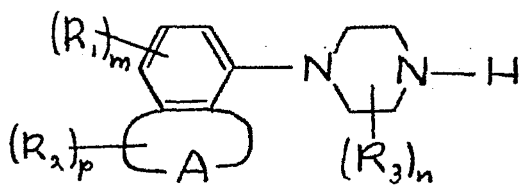

(2)

is reacted with a compound of the formula $L-D-NR_5-CO-R_6$ in which formulae A, $R_1$-$R_3$, $R_5$, D, $R_6$, n, m and p have the meanings given in Claim 1, and L is a leaving group.

7. Method as claimed in Claim 6, characterized in that

a) a compound of formula (3)

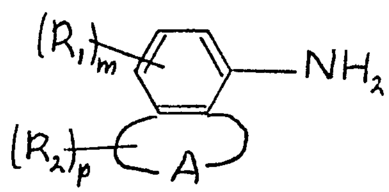

(3)

is reacted with a compound of the formula (4)

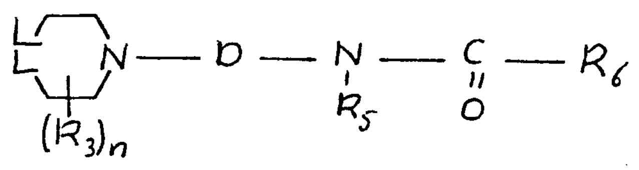

(4)

b) by reacting a compound $L-CH_2-CH_2-L$ with a compound of formula 5

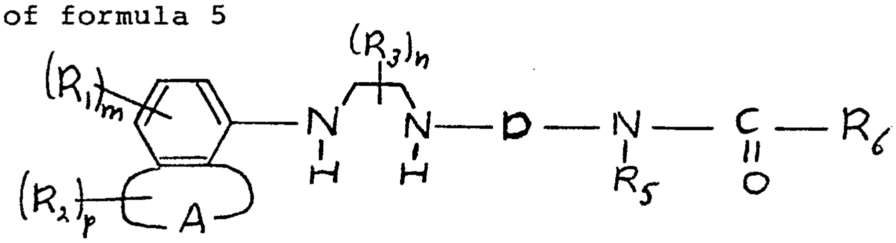

(5)

in which the symbols have the above-mentioned meanings.

8. Method as claimed in Claim 6, characterized in that

a) a compound of formula (6)

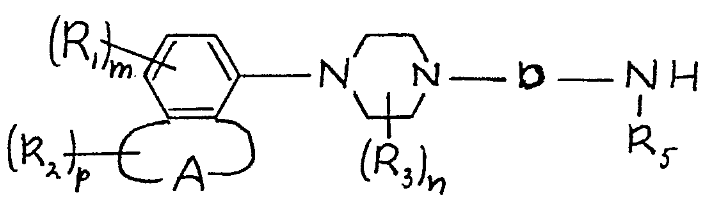

(6)

is reacted with

1) an ester of an acid of the formula $HOOC-R_6$, or
2) an acid chloride of the formula $Cl-CO-R_6$, or
3) a mixed anhydride of the formula $R_4-O-CO-O-CO-R_6$, or
4) a so-called Mukayama ester of formula (7)

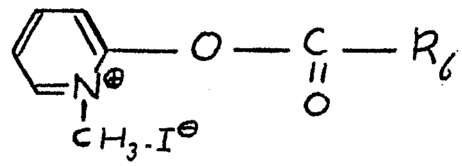

(7)

in which formulae the symbols have the meaning given in Claim 1, and $R_4$ is alkyl having 1-3 carbon atoms; or b) a compound of the formula (8)

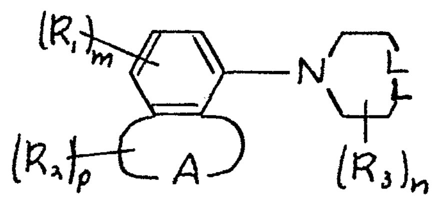

(8)

is reacted with a compound of the formula $NH_2$-D-$NR_5$-CO--$R_6$, wherein the symbols have the meanings given in Claim 1, and L is a leaving group.

9. Method as claimed in Claim 6, characterized in that compounds of formula (1) wherein A is a group -N-$CR_2$=N- or -NH-N=N- and the other symbols have the meaning given in Claim 1, are prepared by reacting a compound of formula (9)

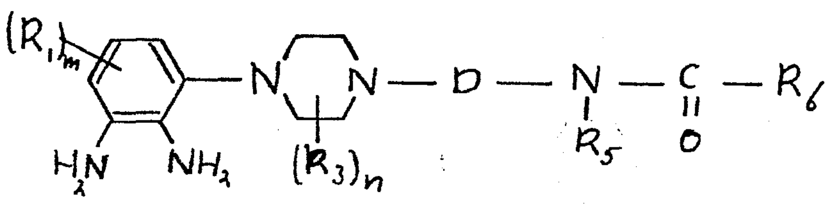

(9)

a) with an ester of an imino acid of formula (10)

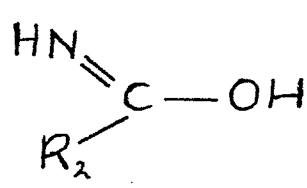

(10)

or

b) with a carboxylic acid of the formula $R_2$-COOH, or

c) with a reactive carbonyl compound, for example N,N'-carbonyldiimidazole, or

d) with an alkali metal nitrite.

10. Method as claimed in Claim 6, characterized in that compounds of formula (1) are prepared by converting or removing one or more protective groups from a corresponding compound.

European Patent Office

# EUROPEAN SEARCH REPORT

0185429

Application number

EP 85 20 2081

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,Y | GB-A-2 097 790 (DELALANDE)<br>* Whole document *<br>--- | 1-10 | C 07 D 319/20<br>C 07 D 263/58<br>C 07 D 249/18<br>A 61 K 31/495 |
| Y | EP-A-0 104 614 (CHUGAI SEIYAKU K.K.)<br>* Whole document *<br>--- | 1-10 | |
| P,X | EP-A-0 138 280 (DUPHAR INTERNATIONAL RESEARCH B.V.)<br>* Whole document *<br>----- | 1-10 | |
| | | | **TECHNICAL FIELDS SEARCHED (Int Cl 4)** |
| | | | C 07 D 319/00<br>C 07 D 263/00<br>C 07 D 249/00 |

The present search report has been drawn up for all claims

| Place of search | Date of completion of the search | Examiner |
|---|---|---|
| THE HAGUE | 25-03-1986 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82